# EUROPÄISCHE PATENTANMELDUNG

(11) **EP 1 614 412 A1**
(43) Veröffentlichungstag der Anmeldung: **11.01.2006**
(21) Anmeldenummer: 05013829.6
(22) Anmeldetag: 18.11.2003
(51) Int. Cl.: A61K 9/18, A61K 31/40

(54) **Verfahren zur Herstellung von freifliessenden, pulverförmigen Atorvastatin-Adsorbaten**

(62) Teilanmeldung aus: 03026546.6
(71) Anmelder: Helm AG, 20097 Hamburg (DE)
(72) Erfinder: Doser, Karl-Heinz, Dr., 21244 Buchholz i.d. Nordheide (DE); Glänzer, Klaus, Dr., 22337 Hamburg (DE); Waldraff, Robert, Dr., 87700 Memingen (DE)
(74) Vertreter: Becker Kurig Straus

(57) **Zusammenfassung**

Die Erfindung betrifft ein Verfahren zur Herstellung von Atorvastatin-Adsorbaten und dessen Solvaten, wobei man von einer, den pharmazeutischen Wirkstoff darin im wesentlichen gelöst enthaltenden Lösung ausgeht, Adsorbermaterial ausgewählt aus der Gruppe der Zellulosen, Cellulosederivate, Polyole, Zucker, Zuckerderivate, Maltodextrine, Cyclodextrine, Stärken, Polydextrosen oder Mischungen daraus, darin suspendiert und das Lösungsmittel durch Trocknen entfernt. Die Erfindung betrifft ebenfalls nach diesem Verfahren herstellbare Atorvastatin-Adsorbate sowie diese enthaltende pharmazeutische Zubereitungen.

## Beschreibung

Die vorliegende Erfindung betrifft ein neuartiges Verfahren zur Herstellung von Atorvastatin-Adsorbaten und von deren Hydraten bzw. Solvaten. Die erfindungsgemäßen Atorvastatin-Adsorbate enthalten in einer besonders bevorzugten Ausführungsform den Wirkstoff in fein verteilter amorpher Form. Besonders bevorzugt liegt der Wirkstoff als Erdalkalisalz, insbesondere als Hemicalcium-Salz sowie als dessen Hydrat-Formen, vor.

Die Erfindung betrifft weiterhin Atorvastatin-Adsorbate und dessen Hydrate bzw. Solvate, die gemäß dem vorgenannten Verfahren herstellbar sind.

Die Erfindung betrifft schließlich auch pharmazeutische Formulierungen, zu deren Herstellung die vorgenannten Atorvastatin-Adsorbate eingesetzt werden. Bevorzugte erfindungsgemäße Arzneiformen sind Tabletten, Kapseln, Pellets und Granulate, die auf an sich bekannte Weise mit üblichen, pharmazeutisch akzeptablen Hilfsstoffen hergestellt werden. Erfindungsgemäß besonders bevorzugt sind den Wirkstoff schnell freisetzende Tabletten, die durch Direktverpressung der erfindungsgemäßen Atorvastatin-Adsorbate hergestellt werden.

Der unter dem INN Atorvastatin bekannte Wirkstoff ist dem Chemiker auch als Calcium-Salz der [R-(R*,R*)]-2-(4-Fluorphenyl)-β,δ-dihydroxy-5-(1-methyl-ethyl)-3-phenyl-phenylamino)-carbonyl]-1H-pyrrol-1-heptansäure geläufig. Dieser Wirkstoff ist ein ausgezeichneter Hemmer des Enzyms 3-Hydroxy-3-methylglutaryl-(Coenzym A)-Reduktase I, auch bekannt unter dem Akronym HMG-CoA-Reduktase; er ist somit als hypolipidämischer und hypocholesterinämischer Wirkstoff zur Therapie von Lipidstoffwechselstörungen verwendbar.

HMG-CoA-Reduktase-Hemmer werden daher auch erfolgreich zur Vorbeugung und zur Behandlung von koronarer Herzerkrankung und anderen Erkrankungen, die auf arteriosklerotischen Gefässveränderungen beruhen, eingesetzt. Über verschiedene Wirkmechanismen kann dabei eine signifikante Lipidsenkung, speziell des Cholesterins, im Plasma erzielt werden (siehe hierzu z.B. Milestones in Drug Therapy / HMG-CoA-Reductase Inhibitors, Hrsg. G. Schmitz und M. Torzewski, Birkhäuser Verlag Basel-Boston-Berlin [2002]).

Die chemische Struktur des Atorvastatins wurde in ihrer racemischen Form erstmals in der EP 247 633 beschrieben. Das Hemicalcium-Salz der als Wirkstoff verwendeten aktiven [R-(R*,R*)]-Form wurde erstmalig in der EP 409 281 offengelegt und als Feststoff beschrieben; diese Schrift enthält aber keine Offenbarung betreffend einer eventuellen Kristallinität des Produktes.

Die Herstellung des Atorvastatins und von wichtigen Zwischenprodukten, wie z. B. der Lacton-Vorstufe des Atorvastatins, wird in verschiedenen Patentschriften erwähnt, so z. B. in EP 330 172, EP 553 213, EP 687 263, EP 915 866, EP 1 054 860, WO 99/57109, WO 01/72706, WO 02/55519.

Aus dem Stand der Technik ist bekannt, dass Atorvastatin in Form seines Hemicalcium-Salzes nicht nur als amorpher Feststoff, wie bereits in der EP 409 281 beschrieben, vorliegt, sondern auch in mehr als 30 kristallinen polymorphen Formen anfallen kann.

So beschreiben die WO 97/03958 sowie die zeitgleich eingereichte WO 97/03959 die kristallinen Formen III sowie I, II und IV des Hemicalcium-Salzes des Atorvastatins. Während die Form III durch Inkubation der Form II für 11 Tage in einer Atmosphäre mit einem Feuchtigkeitsgehalt von 95 % erhalten wird, können die Formen I, II und IV alle aus Methanol bzw. aus Methanol-Wasser-Gemischen in verschiedenen Mischungsverhältnissen und bei unterschiedlichen Temperaturen erhalten werden. Sehr oft kann die gewünschte polymorphe Form gemäß diesen Dokumenten auch nur durch einen massiven Einsatz von Impfkristallen erhalten werden.

Die WO 01/36384 legt die polymorphe Form V des Atorvastatins in Form seines Hemicalcium-Salzes offen, die ebenfalls aus einem Methanol-Wasser-Gemisch erhalten wird.

Die WO 02/57229 beschreibt ebenfalls eine polymorphe Form V des Atovastatins, herstellbar durch Rühren einer Suspension aus rohem Atorvastatin-Hemicalcium in einem Gemisch aus Wasser und absolutem Ethanol im Verhältnis 14:3. Nach den in den Schriften offengelegten Charakterisierungen sind diese beiden Formen aber unterschiedlich.

Die WO 02/41834 beschreibt die polymorphe Form VII des Atorvastatins in Form seines Hemicalcium-Salzes, die durch Rühren einer Suspension der polymorphen Form V oder der polymorphen Form I des Atorvastatins in Form seines Hemicalcium-Salzes in absolutem Ethanol bei Raumtemperatur erhalten werden kann.

Die WO 02/43732 legt die polymorphen Formen VI, VIII, IX, X, XI und XII des Atorvastatins in Form seines Hemicalcium-Salzes offen, wobei die Form VI aus wäßrigem Aceton, die Form VIII aus Ethanol oder aus n-Butanol, die Form IX aus Ethanol oder aus n-Butanol, die Form X aus wäßrigem Ethanol, die Form XI aus Methylethylketon und die Form XII aus wäßrigem Ethanol erhalten werden können. Im Falle gleicher Lösungsmittel ist festzustellen, dass der Erhalt einer bestimmten polymorphen Form von geringfügig geänderten Bedingungen, wie unterschiedlichen Temperaturen oder einem speziellen Wassergehalt abhängig ist.

Die WO 02/51804 benutzt eine zur Beschreibung der polymorphen Formen des Atorvastatins zu den obigen Patentdokumenten unterschiedliche Terminologie. Sie beschreibt die polymorphen Formen X, A, B1, B2, C, D und E des Atorvastatins in Form seines Hemicalcium-Salzes, wobei die Form X aus Methanol und Methyl-t-butylether, die Form A aus Isopropanol, welches Spuren von Wasser enthält, die Form B1 aus einem Acetonitril-THF-Gemisch, die Form B2 aus reinem Acetonitril, die Form C aus wäßrigem Isopropanol, die Form D aus Ethanol und die Form E aus Methylethylketon durch Ausfällen mit Heptan erhalten werden können. Auch hier ist wieder festzustellen, dass bei gleichen Lösungsmitteln geringfügige Änderungen bei den sonstigen Bedingungen schon zur Kristallisation einer anderen polymorphen Form führen.

Die WO 03/04470 beschreibt schließlich die polymorphen Formen V, VI, VII, VI1I, IX, X, XI, XII, XIII, XIV, XV, XVI, XVII, XVIII und XIX des Atorvastatins in Form seines Hemicalcium-Salzes, wobei die Form V, die als Trihydrat charakterisiert ist, aus wäßrigem Acetonitril, die Form VI aus wäßrigem DMF, die Form VII, die als Sesquihydrat charakterisiert wird, aus wäßrigem Aceton, die Form VIII, bei der es sich um ein Dihydrat handelt, wieder aus wäßrigem DMF, und die Form IX wieder aus wäßrigem Aceton erhalten wird. Die Form X, bei der es sich um ein Trihydrat handelt, ist aus wäßrigem Isopropanol erhältlich, die Form XI wieder aus wäßrigem Acetonitril, die Form XII aus THF-haltigem Wasser, die Form XIII aus wäßrigem Methanol und die Form XIV, bei der es sich um ein Hexahydrat handelt, wieder aus wäßrigem Isopropanol. Die Form XV, beschrieben als Trihydrat, fällt aus wäßrigem Aceton an, die Form XVI, die als Tetrahydrat-Acetonitril-Solvat beschrieben wird, aus wäßrigem Acetonitril. Die Form XVIII, bei der es sich um ein Solvat handelt, aus einem DMF-Wasser-Acetonitril-Gemisch und die Form XIX, bei der es sich ebenfalls um ein Solvat handelt, aus Methylethylketon. Die Form XVII, bei der es sich um ein Tetrahydrat handelt, schließlich wird durch längeres Trocknen der Form XVI erhalten. Auch aus dieser Offenlegung wird wieder deutlich, dass geringfügige Änderungen der Kristallisationsbedingungen zu anderen polymorphen Formen bei der Verwendung gleicher Lösungsmittelsysteme führen können. Es ist auch bei diesem Patentdokument festzustellen, wie bei den oben zitierten Offenlegungen zur Kristallisation des Atorvastatin-Hemicalcium-Salzes, dass die Entstehung von definierten polymorphen Formen auch von dem Ausgangspolymorph, eventuell auch von Impfkristallen und von den unterschiedlichen Trocknungsbedingungen stark abhängig ist.

Zusammenfassend kann daher festgestellt werden, dass die Kristallisation von Atorvastatin-Hemicalcium in einer definierten polymorphen Form außerordentlich stark von geringfügig geänderten Prozessparametern abhängig ist, was zu einer sehr aufwendigen Prozesssteuerung führt, da ein solches definiertes Polymorph für einen pharmazeutischen Wirkstoff unbedingt sicherzustellen ist, um den regulatorischen Anforderungen an Arzneimittel gerecht zu werden und natürlich auch um die gleichbleibende Qualität des Arzneimittels und damit die Einnahmesicherheit für den Patienten zu gewährleisten.

Eine Möglichkeit, dieses Problem zu lösen und zu einem vorteilhafteren Isolationsprozess zu kommen, ist die Verwendung von amorphem Atorvastatin-Hemicalcium. Zur Gewinnung der amorphen Form sind im Stand der Technik mehrere Verfahren beschrieben, so z. B. in der EP 839 132: aus einer Lösung des Wirkstoffes in einem THF-Toluol-Gemisch durch Entfernen des Lösungsmittels, in der EP 1 185 264: aus einer Lösung des Wirkstoffes in THF durch Ausfällen des Produktes mit Heptan oder gemäß der EP 1 235 800: durch "Umkristallisation" des Wirkstoffes aus niederen Alkoholen.

Die Verwendung von amorphem Atorvastatin-Hemicalcium als Wirkstoff in pharmazeutischen Formulierungen bringt zum einen das Problem der im Vergleich zu kristallinem Wirkstoff geringeren Stabilität der amorphen Form gegenüber Umfeldbedingungen, wie Sauerstoff, Licht, Wärme und Restfeuchtigkeit, sowie der höheren Empfindlichkeit in Bezug auf stabilitätsmindemde Wechselwirkungen mit pharmazeutischen Hilfs- und Zusatzstoffen mit sich. So ist z. B. bekannt, dass sich Atorvastatin-Hemicalcium in Gegenwart von Feuchtigkeit und leicht sauer reagierenden Substanzen sehr leicht in das Atorvastatin-Lacton umwandelt, was zu einer nicht akzeptablen Wirkstoffabnahme in der fertigen Tablette führen kann. Um den Einfluss derartiger Reaktionen in der fertigen Arzneiform zu verhindern, wurden meist alkalisierende Zusatzstoffe wie etwa bestimmte Alkali- und Erdalkaliverbindungen, alkalische Puffersysteme (siehe z. B. US 5,180,589, US 5,686,104, WO 00/35425 und WO 01/93859) oder auch die Verwendung polymerer Verbindungen mit Amido- oder Aminogruppen (Polyvinylpyrrolidone oder Cholestyramine, siehe WO 01/76566) zur Stabilisierung vorgeschlagen. Eine Kombination beider Stabilisierungsarten (Natriumhydroxid plus Polyvinylpyrrolidon) wurde für analoge Statin-Verbindungen ebenfalls verwendet (WO 98/57917).

Ein weiteres Problem der Verwendung von amorphem Atorvastatin-Hemicalcium als Wirkstoff, ist die Tatsache, dass nach den experimentellen Erfahrungen der Erfinder der vorliegenden Anmeldung beim Fällunsgprozess der amorphen Form öfter auch heterogene Produkte: ein Teil kristallin und ein anderer Teil amorph, erhalten werden, was dazu führt, dass der Fällungsprozess wiederholt werden muss. Jede zusätzliche Herstellungsschritt birgt aber das Risiko eines Substanzverlustes von ca. 5 % bis 10 % in sich, was aus ökonomischer Sicht für Wirkstoffe wie Atorvastatin-Hemicalcium, das in einer langwierigen, aufwendigen und teuren Synthesefolge hergestellt werden muss, sicher nicht erwünscht ist.

Aus der DE 10008506 A1 ist zwar für einen analogen Statin-Wirkstoff , nämlich Cerivastatin, ein Verfahren zur Herstellung eines Wirkstoffgranulats bekannt, das einen solchen Substanzverlust vermeidet, wobei eine ausschließlich wäßrige Wirkstofflösung in Gegenwart eines Füllstoffes und eines Bindemittels direkt granuliert wird und das so erhaltene Granulat nach dem Trocknen in Tabletten weiter verarbeitet wird. Für das im vorliegenden Fall bestehende Problem hinsichtlich des Wirkstoffs Atorvastatin-Hemicalcium ist dies aber keine technisch durchführbare Lösung, da in den in DE 10008506 A1 beschriebenen Granulaten der Wirkstoff in einer maximalen Menge von 0,5 % (w/w) vorliegt. Für therapeutische Dosierungen des Atorvastatin-Hemicalciums muss der Wirkstoffgehalt aber zwischen 20 mg und 80 mg liegen. Dies würde gemäß der Offenbarung der DE 10008506 A1 zu Tablettengewichten zwischen 4 und 16 g führen. Der akzeptable Bereich für Tabletten, die zur oralen Einnahme geeignet sind, d. h. vom Patienten geschluckt werden müssen, liegt zwischen 100 mg und 1 g.

Die bekannten Verfahren zur Herstellung von pharmazeutischen Formulierungen des Atorvastatin-Hemicalciums sind, soweit sie überhaupt durchführbar sind, technisch daher sehr aufwendig, zeit- und kostenintensiv und lösen das Problem einer stabilen Arzneiform bislang nicht oder nicht zufriedenstellend. Letzteres trifft ganz besonders auf das wegen seiner besseren Herstellbarkeit und wegen guter Auflöseeigenschaften bevorzugte Atorvastatin-Hemicalcium mit großer spezifischer Oberfläche, d. h. insbesondere amorph oder feinpulvrig, zu. In solchen Fällen reichen die bekannten, zuvor ausgeführten Stabilisierungen des Standes der Technik nicht aus.

Daher ist es die Aufgabe der vorliegenden Erfindung, ein einfaches und kostengünstiges Verfahren zur Herstellung von stabilen Atorvastatin-Pulversystemen zu entwickeln, die direkt zur Herstellung von pharmazeutischen Formulierungen eingesetzt werden können, wobei dieses Verfahren jedoch nicht auf eine besonders bevorzugte Wirkstoffmorphologie eingeschränkt ist, welches die oben diskutierten Nachteile vermeidet.

Diese Aufgabe wird erfindungsgemäß durch ein Verfahren zur Herstellung von Atorvastatin-Adsorbaten gemäß Anspruch 1 gelöst. Dabei geht man von einer Lösung des Atorvastatins oder seiner Salze in einem vorwiegend organischen Lösungsmittel aus, wobei der Wirkstoff in den organischen Lösungsmitteln gelöst vorliegt, suspendiert darin das Adsorbermaterial und entfernt das Lösungsmittel durch Trocknen. Der Gesamtwassergehalt des Lösungsmittels beträgt erfindungsgemäß nicht mehr als 10 Vol.-%, bevorzugt nicht mehr als 5 Vol.-% (vergleiche Anspruch 1).

In einer bevorzugten Ausführungsform der Erfindung enthalten die Atorvastatin-Adsorbate den Wirkstoff in fein verteilter amorpher Form, insbesondere als Calciumsalz. Das amorphe Atorvastatin gemäß der vorliegenden Erfindung kann sowohl in wasserfreier Form als auch in Form von Solvaten bzw. Hydraten vorliegen.

Die Erfindung betrifft weiterhin nach dem vorgenannten Verfahren herstellbare Atorvastatin-Adsorbate und dessen Solvate bzw. Hydrate. Die Erfindung betrifft weiterhin pharmazeutische Formulierungen enthaltend diese neuen Atorvastatin-Adsorbate. Die pharmazeutischen Formulierungen enthalten gegebenenfalls weitere Hilfsstoffe und können in die gewünschte Darreichungsform überführt werden. Besonders bevorzugt sind den Wirkstoff schnell freisetzende, durch Direktverpressung hergestellte Tabletten.

Für das erfindungsgemäße Verfahren zur Herstellung der Atorvastatin-Adsorbate eignen sich organische Lösungsmittel für die den pharmazeutischen Wirkstoff enthaltende Lösung. Die organischen Lösungsmittel sind insbesondere ausgewählt aus der Gruppe der niederen Alkanole mit 1 bis 4 Kohlenstoffatomen, der Gruppe der Ether und der Gruppe der aliphatischen Ketone und Gemische der vorgenannten Lösungsmittel. Besonders bevorzugt sind Methanol, Ethanol, Isoproponol, n-Propanol, Aceton und andere Lösungsmittel wie Ethylacetat, Methylethylketon, MTBE (Methyltert.-butylester) und Gemische aus Ethylacetat und Hexan sowie Gemische der vorgenannten Lösungsmittel.

Als Absorbermaterialien werden erfindungsgemäß pharmazeutisch akzeptable Hilfsstoffe eingesetzt, die für eine schnelle Wirkstofffreisetzung geeignet sind, wie Cellulosen und Cellulosederivate, insbesondere mikrokristalline Cellulose, Polyole, insbesondere Mannit, Zucker und Zuckerderivate, insbesondere Lactose, Maltodextrin, Stärken, Cyclodextrine, Polydextrosen oder Gemische der vorgenannten Substanzen. Mikrokristalline Cellulose, Lactose und Mannit sind erfindungsgemäß bevorzugt.

Das Verhältnis des pharmazeutischen Wirkstoffes zum Adsorbermaterial liegt erfindungsgemäß im Bereich von 1:0,1 bis 10, wobei insbesondere ein Bereich von 1:1 bis 1:2 bevorzugt wird.

Für die Herstellung der pharmazeutischen Formulierungen, wobei insbesondere Tabletten bevorzugt sind, können alle üblichen pharmazeutischen Hilfsstoffe verwendet werden. Als Füllstoffe können z.B. Cellulosen und Cellulosederivate (z.B. mikrokristalline Cellulose, native Cellulose, Hydroxypropylcellulose, Hydroxypropylmethylcellulose, Methylcellulose), Zucker (z. B. Lactose, Fructose, Saccharose, Glukose, Maltose), Zuckeralkohole (z.B. Lactit Mannit, Sorbit, Xylit) anorganische Füllstoffe (z.B. Calciumphosphate und Calciumsulfate) und Stärken (z.B. Maisstärke, Kartoffelstärke, Weizenstärke, Dextrine, pregelatinisierte Stärken) verwendet werden. Darüber hinaus können alle weiteren Hilfsstoffe, die dem Fachmann aufgrund seines galenischen Basiswissens bekannt sind, wie z.B. Schmiermittel, Zerfallshilfsmittel, Netzmittel, alkalische Zusatzstoffe, Stabilisatoren sowie Aromen, Farbpigmente und Farbstoffe zur Herstellung der erfindungsgemässen Arzneimittelformulierungen eingesetzt werden.

Der Anteil an Bindemittel in der Gesamtmischung der Arzneimittelzubereitung ist bevorzugt 0 bis 20% (m/m), der Anteil an Füll- und Hilfsstoffen in der Gesamtmischung beträgt 20 bis 99%, bevorzugt von 50 bis 99%.

Mit dem erfindungsgemäßen Verfahren werden überraschend stabile Adsorbate von Atorvastatin, insbesondere von amorphem Atorvastatin, hergestellt. Diese Atorvastatin-Adsorbate werden als pharmazeutischer Wirkstoff in den erfindungsgemäßen Zubereitungen eingesetzt. Bevorzugt wird Atorvastatin im Rahmen dieser Erfindung in Form seiner Salze eingesetzt. Beispiele der erfindungsgemäß verwendeten Atorvastatin-Salze sind das Mononatrium- und die Monokalium-Salze sowie die Magnesium- und Calcium-Salze sowie deren Hydrate und Solvate. Ganz besonders bevorzugt sind das Atorvastatin-Hemicalcium-Salz und dessen Hydrate und Solvate.

Im Rahmen dieser Erfindung können weiterhin die jeweiligen Hydrate, Solvate, Ester, Lactone und Tautomere des Atorvastatins eingesetzt werden, die insbesondere im Rahmen der Wirkstoffherstellung am Ende des Syntheseweges in Lösung anfallen können, was die Isolation des reinen Wirkstoffes vermeidet.

Es wurde nun erfindungsgemäß ein Verfahren gefunden, das ausgehend von einer Lösung des Atorvastatins oder eines seiner Salze, Hydrate, Solvate, Ester, Lactone und Tautomere in einem organischen Lösungsmittel zu direkt weiterverarbeitbaren Wirkstoff-Absorbaten führt.

Die Atorvastatin-Wirkstofflösung kann grundsätzlich in einer Ausführungsform der Erfindung durch Auflösen des Atorvastatins oder eines seiner Salze, Hydrate, Solvate, Ester, Lactone und Tautomere in einem geeigneten organischen Lösungsmittel hergestellt werden; vorteilhafter ist jedoch, eine im Rahmen der Synthese ohnehin anfallende Wirkstofflösung ohne Isolierung des Atorvastatins direkt zu verwenden.

Beispielsweise kann das Atorvastatin gemäß der EP 409 281 A1, Beispiel 10 hergestellt werden, wobei dann auf den Rekristallisierungsschritt durch Lösen in Ethylacetat und Ausfällen mit Hexan verzichtet wird, und statt dessen das Adsorbermaterial in der Wirkstofflösung suspendiert und das Lösungsmittel durch Trocknen später entfernt wird. Die Art des organischen Lösungsmittels ergibt sich dann im Einzelfall aus dem abschließenden Synthese-Schritt der Wirkstoffherstellung.

Zu dieser organischen Wirkstofflösung wird ein darin nicht bzw. gering löslicher pharmazeutisch akzeptabler Hilfsstoff als Adsorbermaterial zugegeben, gut benetzt und unmittelbar danach das Lösungsmittel durch Trocknen entfernt. Der Trocknungsvorgang kann durch Temperieren und Vakuum unterstützt werden. Er wird vorteilhaft so gestaltet, dass durch geeigneten mechanischen Einfluss (Dreh-, Taumel-, Rührbewegung) eine einheitliche Verteilung gegeben ist. Das Lösungsmittel kann durch Arbeiten im geschlossenen System zurückgewonnen und für den Folgeprozeß wieder eingesetzt werden. Erfindungsgemäß ist, dass eine Ausfällung und Isolierung des Atorvastatins entfällt. Gemäß dem beschriebenen Verfahren hergestellte atorvastatinhaltige Adsorbate können direkt zur Weiterverarbeitung zu Arzneiformen wie Tabletten, Kapseln, Pellets oder Granulaten eingesetzt werden, bevorzugt zur Weiterverarbeitung mittels eines Direktkompressionsverfahrens.

Optional können die so erhaltenen Adsorbate oder Arzneiformen für spezielle Anwendungen weiter mit Überzügen aus pharmazeutischen Polymethacrylaten, wie z. B. Eudragit®-Filme, Methylcellulose, Ethylcellulosen, Hydroxypropylmethylcellulosen, Celluloseacetatphthalaten und/oder Schellack versehen werden, um einen bestimmten Anwendungszweck, z. B. kontrollierte Wirkstoff-Freisetzung, Geschmacksabdeckung zu erzielen. Hierfür stehen dem pharmazeutischen Fachmann ausreichende technische Möglichkeiten zur Verfügung.

Überraschend wurde gefunden, dass nach dem erfindungsgemäßen Verfahren hergestellte Adsorbate den Wirkstoff binden ohne dass es zur Ausbildung von wirkstofftypischen Kristallstrukturen kommt. Dies konnte anhand von röntgendiffraktometrischen Messungen gezeigt werden. Vergleichende Stabilitätsuntersuchungen zeigen zudem, dass die bevorzugten Atorvastatin-Adsorbate eine bessere Stabilität und eine schnellere Auflösegeschwindigkeit aufweisen, als der reine amorphe Wirkstoff. Bestimmte Adsorbermaterialien, z. B. auf Siliziumdioxidbasis weisen stärkere Bindungseigenschaften und damit ein anderes Freisetzungsverhalten des adsorbierten Atorvastatins auf.

Die genannten Eigenschaften bleiben auch insbesondere dann erhalten, wenn die Atorvastatin-Adsorbate zu Arzneiformen, wie z. B. Tabletten verarbeitet werden.

Die Erfindung wird nun anhand der Abbildungen und Beispiele näher erläutert, ohne jedoch die Erfindung darauf zu beschränken.

Es zeigen,
- Abbildung 1: ein Pulverröntgenbeugungsdiagramm eines erfindungsgemäßen AtorvastatinMikrokristalline Cellulose-Adsorbates (im Verhältnis 1:1) in der oberen Kurve, sowie als Vergleich in der unteren Kurve mikrokristalline Cellulose alleine,
- Abbildung 2: ein Pulverröntgenbeugungsdiagramm eines erfindungsgemäßen AtorvastatinMannitol-Adsorbates (im Verhältnis 1:1) in der oberen Kurve, sowie als Vergleich in der unteren Kurve Mannitol alleine,
- Abbildung 3: ein Pulverröntgenbeugungsdiagramm eines erfindungsgemäßen AtorvastatinLactose-Adsorbates (im Verhältnis 1:1) in der oberen Kurve, sowie als Vergleich in der unteren Kurve Lactose alleine,
- Abbildung 4: ein Pulverröntgenbeugungsdiagramm eines erfindungsgemäßen AtorvastatinMikrokristalline Cellulose-Adsorbates (im Verhältnis 1:1,5) in der oberen Kurve, sowie als Vergleich in der unteren Kurve mikrokristalline Cellulose alleine,
- Abbildung 5: ein Pulverröntgenbeugungsdiagramm eines erfindungsgemäßen AtorvastatinMannitol-Adsorbates (im Verhältnis 1:1) in der oberen Kurve, sowie als Vergleich in der unteren Kurve Mannitol alleine,
- Abbildung 6: ein Pulverröntgenbeugungsdiagramm eines erfindungsgemäßen AtorvastatinMikrokristalline Cellulose-Adsorbates (im Verhältnis 1:2), in der oberen Kurve, sowie als Vergleich in der unteren Kurve mikrokristalline Cellulose alleine,

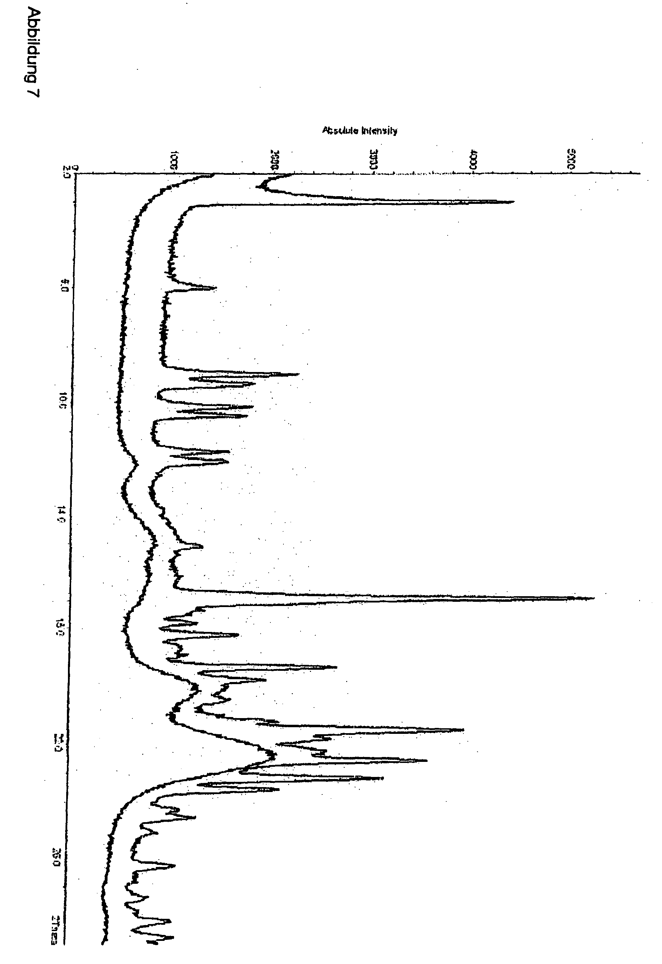

- Abbildung 7: ein Pulverröntgenbeugungsdiagramm von kristallinem Atorvastatin (sogenannte Form I, vergleiche WO 97/03959 Al), im Pulvermischungsverhältnis 1:1 mit mikrokristalliner Cellulose, sowie als Vergleich in der unteren Kurve mikrokristalline Cellulose alleine,

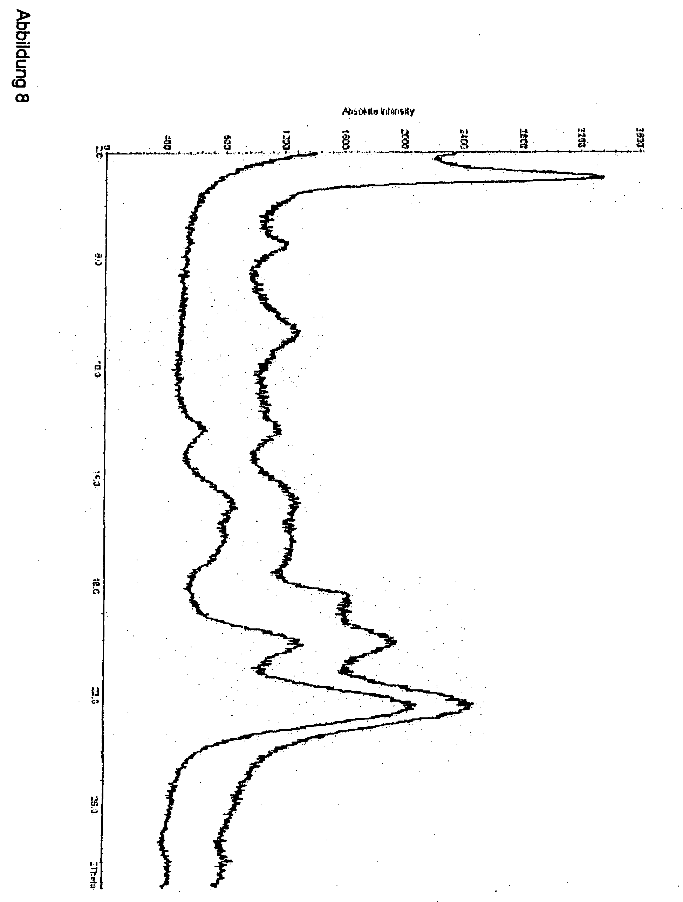

- Abbildung 8: ein Pulverröntgenbeugungsdiagramm von kristallinem Atorvastatin (sogenannte Form V, vergleiche WO 01/36384 Al), im Pulvermischungsverhältnis 1:1 mit mikrokristalliner Cellulose, sowie als Vergleich in der unteren Kurve mikrokristalline Cellulose alleine.

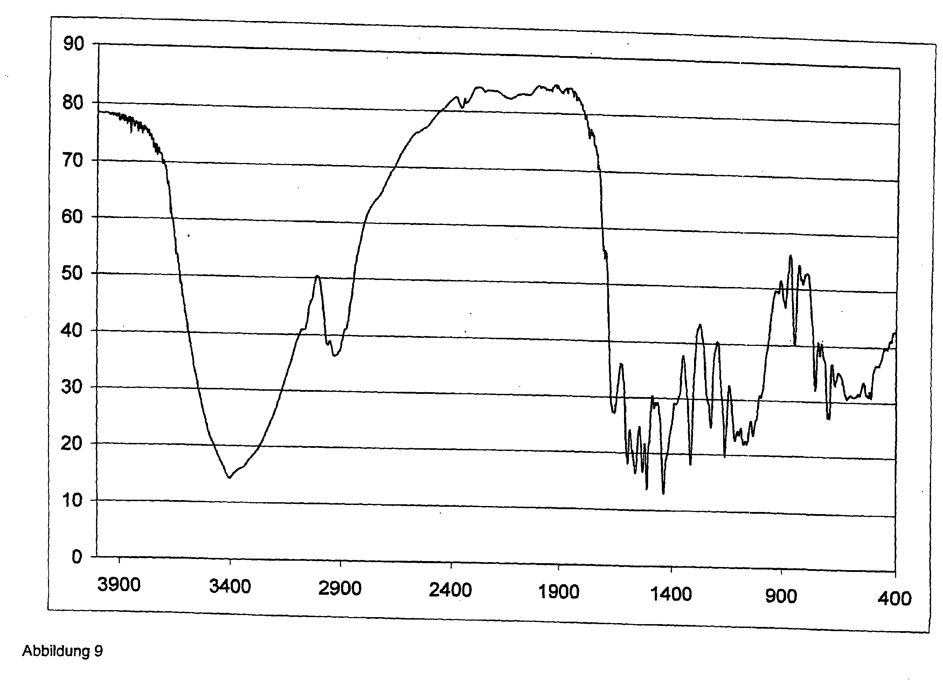

- Abbildung 9: FTIR-Spektrum eines erfindungsgemäßen Atorvastatin-Mikrokristalline Cellulose Adsorbates (im Verhältnis 1:1),

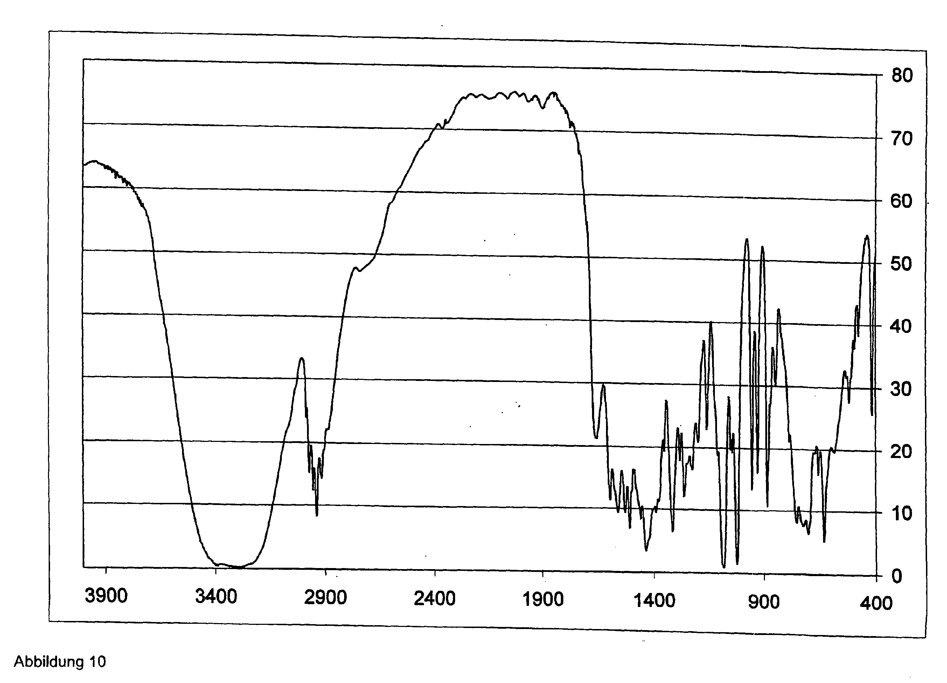

- Abbildung 10: FTIR-Spektrum eines erfindungsgemäßen Atorvastatin-Mannitol-Adsorbates (im Verhältnis 1:1) und

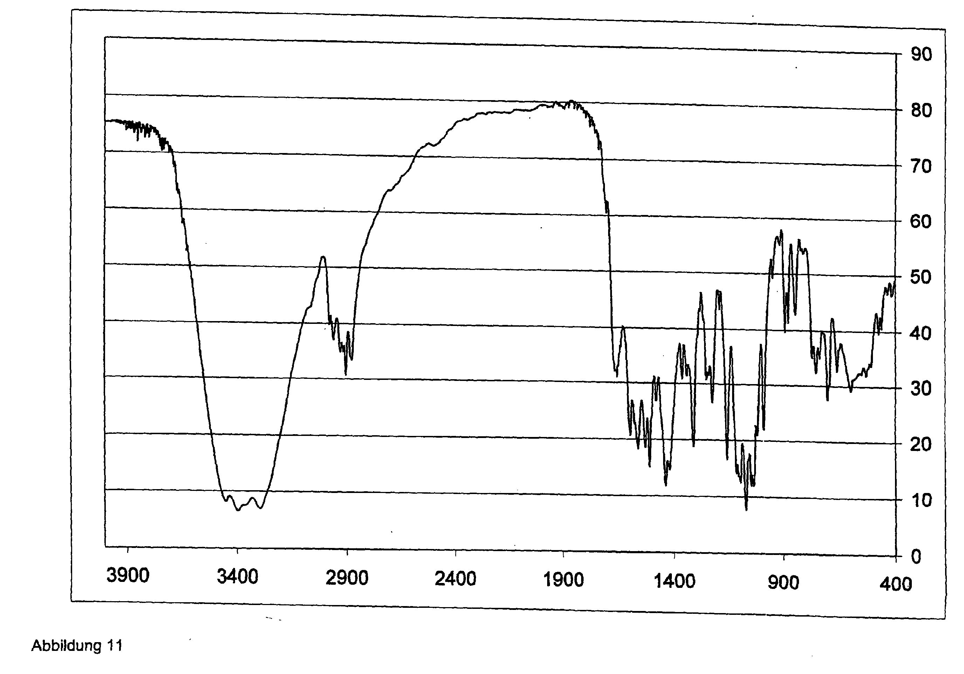

- Abbildung 11: FTIR-Spektrum eines erfindungsgemäßen Atorvastatin-Lactose-Adsorbates (im Verhältnis 1:1).

### Beispiele 1 bis 6

Verwendete apperative Ausstattung der analytischen Untersuchungen:

### HPLC-Messungen:

Alle HPLC-Messungen wurden mit einem Agilent 100-HPLC durchgeführt.

| | |
|---|---|
| Verwendete Säule | Inertril ODS 2,5µ (150 x 4,6 mm) |
| Mobile Phase | 55% 0,05M Natriumacetat |
| | 54% Acetonitril |
| | pH: 4,0 (Einstellung mit Essigsäure) |
| Fliessgeschwindigkeit | 1 ml min⁻¹ |
| Detektor | UV bei 246 nm |
| Injektionsvolumen | 20 ml |
| Retentionszeit Atorvastatin | ca. 15 min |
| Analysedauer | 60 min |

### Röntgenmessungen:

Alle Pulverröntgenbeugungsdiagramme wurden wie folgt gemessen:

| | |
|---|---|
| Gerät | STADI P Transmissions-Diffraktometer |
| | Cu-Ka₁-Strahlung (1= 1.54056Å), U = 40 kV, I = 35 mA |
| | Primärstrahl-Monochromator (gekrümmt Ge 111) |
| Detektor | Linearstellungssensitiv |
| Spaltbreite | 1 mm |
| Linear PSD | 20 = 2° bis 34°, 25 s / 0,2° stufenweise, Inkrement Δ2Θ = 0,02 |
| Probe | Pulver in Mylar-Folie |

### IP-Spektren:

| | |
|---|---|
| Gerät | GENESIS II FTIR-Spektrometer |
| Messmethode | KBr-Pressling mit 1% Testsubstanz |

Die Spektren sind als Transmissionswerte (in %) in Abhängigkeit von den Wellenzahlen (cm⁻¹) dargestellt.

### Beispiel 1: Atorvastatin-Mikrokristalline Cellulose-Adsorbat

Zu einer Lösung von heterogenem Atorvastatin-Hemicalcium in Aceton (0,15g/ml) werden 0,15 g/ml mikrokristalline Cellulose (CelphereSCP-100®) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

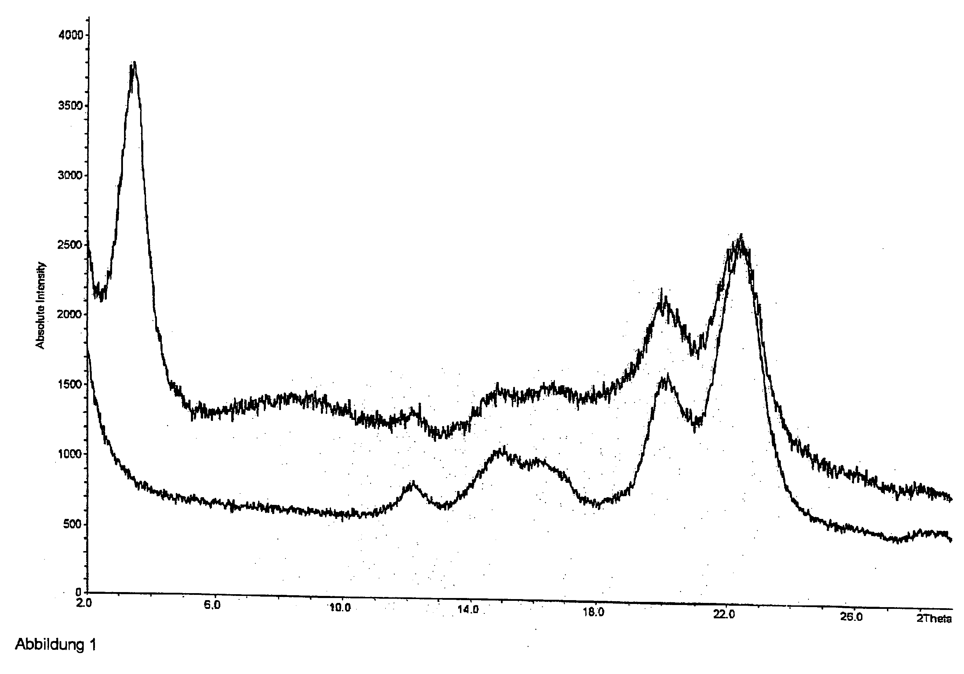

Wirkstoffgehalt des Adsorbates mittels HPLC: 49,6 % (theoretisch 50%) Pulverröntgenbeugungsdiagramm: Abbildung 1

Verunreinigungsprofil: Summe aller Verunreinigungen: HPLC, in %:

| | **Ausgang** | **15 Tage (70°C / 75% relative Feuchtigkeit)** |
|---|---|---|
| **Probe (Adsorbat)** | **0,76** | **1,11** |
| **Vergleich (amorphes Atorvastatin-Calcium)** | **1,07** | **1,92** |
| **Tablette** | **0,77** | **1,07** |

Aus dem Adsorbat wurden Atorvastatin-Tabletten mittels Direktverpressung nach folgender Zusammensetzung hergestellt:
■ Atorvastatin-Mikrokristalline-Cellulose-Adsorbat 80 mg
■ Mikrokristalline Cellulose (CelphereSCP-100®) 408 mg
■ Hilfsstoffe (Croscarmellose-Natrium, Natriumlaurylsulfat,
   Siliziumdioxid, Magnesiumstearat) in den üblichen Mengen 72 mg

Die verwendeten Mengen der weiteren Hilfsstoffe sind dem Fachmann aufgrund seines Basiswissens bekannt und können Standardwerken zur Formulierung von Tabletten, wie z.B. Ritschel et al., die Tablette, Editio Cantor - Aulendorf, 2. Auflage [2002] entnommen werden.

Eigenschaften der pressfertigen Mischung und der Tablette:
■ Kompressibilität und Fließfähigkeit: gut
■ Mittlere Härte 142 N
■ Abrieb: 0,06% (bestimmt nach Ph. Eur.)
■ Zerfallzeit: 40 sek. (bestimmt nach Ph. Eur.)
■ Freisetzung: 100% nach 5 Min. (Ph. Eur., 1000 ml
   Wasser, 37°C, Paddel 75 U min⁻¹)

Die so erhaltenen Tabletten können gegebenenfalls mit einem Überzug versehen werden.

### Beispiel 2: Atorvastatin-Mannitol-Adsorbat

Zu einer Lösung von heterogenem Atorvastatin-Hemicalcium in Aceton (0,15 g/ml) werden 0,15g/ml Mannitol *(Mannogern®*) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

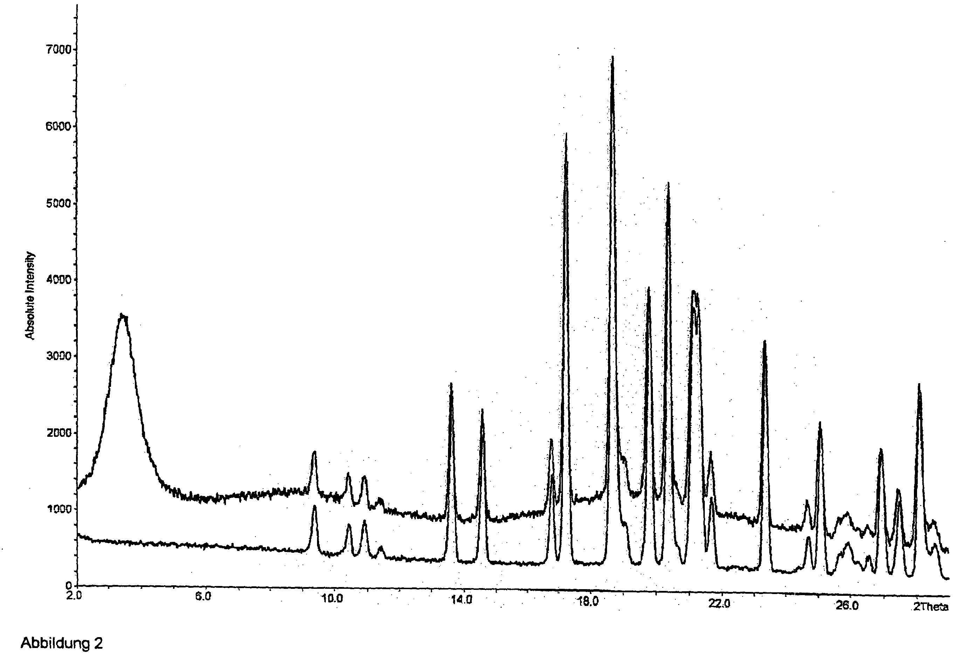

Wirkstoffgehalt des Absorbates, mittels HPLC: 49,85% (theoretisch 50%)
Pulverröntgenbeugungsdiagramm: Abbildung 2
Verunreinigungsprofil (Summe aller Verunreinigungen, HPLC, in %):

| | **Ausgang** | **15 Tage (70°C / 75% relative Feuchtigkeit)** |
|---|---|---|
| **Probe (Adsorbat)** | **0,91** | **1,40** |
| **Vergleich (amorphes Atorvastatin-Calcium)** | **1,07** | **1,92** |
| **Tablette** | **0,85** | **1,01** |

Aus dem Adsorbat wurden Atorvastatin-Tabletten mittels Direktverpressung nach folgender Zusammensetzung hergestellt:
■ Atorvastatin-Mannitol-Adsorbat 80 mg
■ Mannitol 408 mg
■ Hilfsstoffe (wie in Bsp. 1) 72 mg

Eigenschaften der pressfertigen Mischung und der Tabletten:
■ Kompressibilität und Fließfähigkeit: befriedigend bis gut
■ Mittlere Härte 153 N
■ Abrieb: 0,18% (bestimmt nach Ph. Eur.)
■ Zerfallzeit: 65 sek. (bestimmt nach Ph. Eur.)
■ Freisetzung: 100% nach 7 Min. (Ph. Eur., 1000 ml
   Wasser, 37°C, Paddel 75 U min⁻¹)
   Die so erhaltenen Tabletten können gegebenenfalls mit einem Überzug versehen werden.

### Beispiel 3: Atorvastatin-Lactose-Adsorbat

Zu einer Lösung von heterogenem Atorvastatin-Hemicalcium in Aceton (0,15 g/ml) werden 0,15g/ml Lactose (Lactopress® Anhydrous) zugegeben und gleichmäßig suspendiert. Das Lösungsmittel wird nun unter permanenter Bewegung und Vakuum (Rotationsverdampfer oder Trommeltrockner) bei 25°C abgetrocknet. Die Mischung wird abschließend zur Entfernung von Restlösemittel kurzzeitig bei 35°C nachgetrocknet.

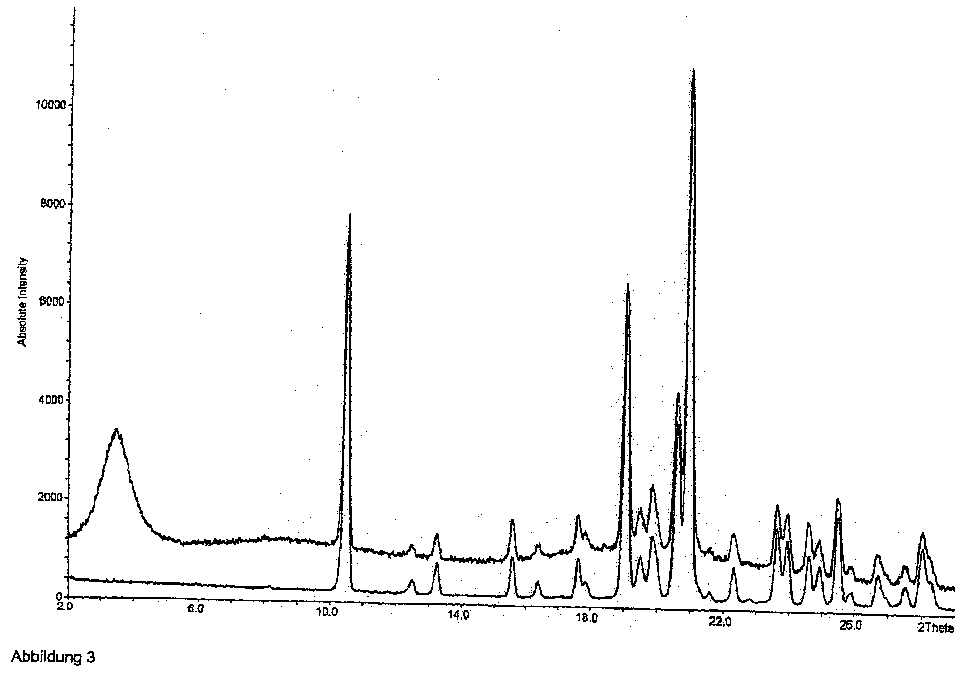

Wirkstoffgehalt des Absorbates mittels HPLC: 51,18% (51,18% (theoretisch 50%)
Pulverröntgenbeugungsdiagramm: Abbildung 3
Verunreinigungsprofil (Summe aller Verunreinigungen, HPLC, in %):

| | **Ausgang** | **15 Tage (70°C / 75% relative Feuchtigkeit)** |
|---|---|---|
| **Probe (Adsorbat)** | **0,80** | **1,03** |
| **Vergleich (amorphes Atorvastatin-Calcium)** | **1,07** | **1,92** |
| **Tablette** | **0,79** | **1,05** |

Aus dem Adsorbat wurden Atorvastatin- Tabletten mittels Direktverpressung nach folgender Zusammensetzung hergestellt:
■ Atorvastatin-Lactose-Adsorbat 80 mg
■ Lactose 408 mg
■ Hilfsstoffe (wie in Bsp. 1) 72 mg

Eigenschaften der pressfertigen Mischung und der Tabletten:
■ Kompressibilität und Fließfähigkeit: befriedigend bis gut
■ Mittlere Härte 138 N
■ Abrieb: 0,18% (bestimmt nach Ph. Eur.)
■ Zerfallzeit: 80 sek. (bestimmt nach Ph. Eur.)
■ Freisetzung: 100% nach 8 Min. (Ph. Eur., 1000 ml
   Wasser, 37°C, Paddel 75 U min⁻¹)

Die so erhaltenen Tabletten können ebenfalls mit einem Überzug versehen werden.

### Beispiel 4: Atorvastatin-Mikrokristalline-Cellulose-Adsorbat

Zu einer Lösung von heterogenem Atorvastatin-Hemicalcium in Ethanol (0,15 g/ml) werden 0,225g/ml mikrokristalline Cellulose (Verhältnis Wirkstoff : Adsorbat 2:3) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

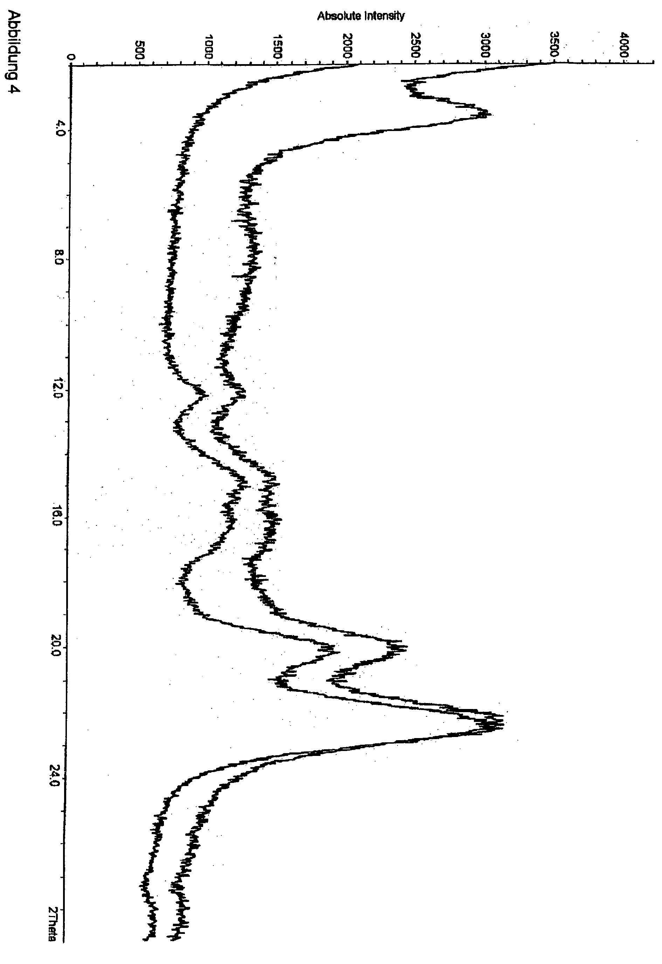

Wirkstoffgehalt des Absorbates mittels HPLC: 40,3%: (theoretisch 40%)
Pulverröntgenbeugungsdiagramm: Abbildung 4
Verunreinigungsprofil (Summe aller Verunreinigungen, HPLC, in %):

| | **Ausgang** | **15 Tage (70°C / 75% relative Feuchtigkeit)** |
|---|---|---|
| **Probe (Adsorbat)** | **0,83** | **1,04** |
| **Vergleich (amorphes Atorvastatin-Calcium)** | **1,07** | **1,92** |

### Beispiel 5: Atorvastatin-Mannitol-Adsorbat

Zu einer Lösung von heterogenem Atorvastatin-Hemicalcium in Ethanol (0,15 g/ml) werden 0,15 g/ml Mannitol (1:1 Mischung) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

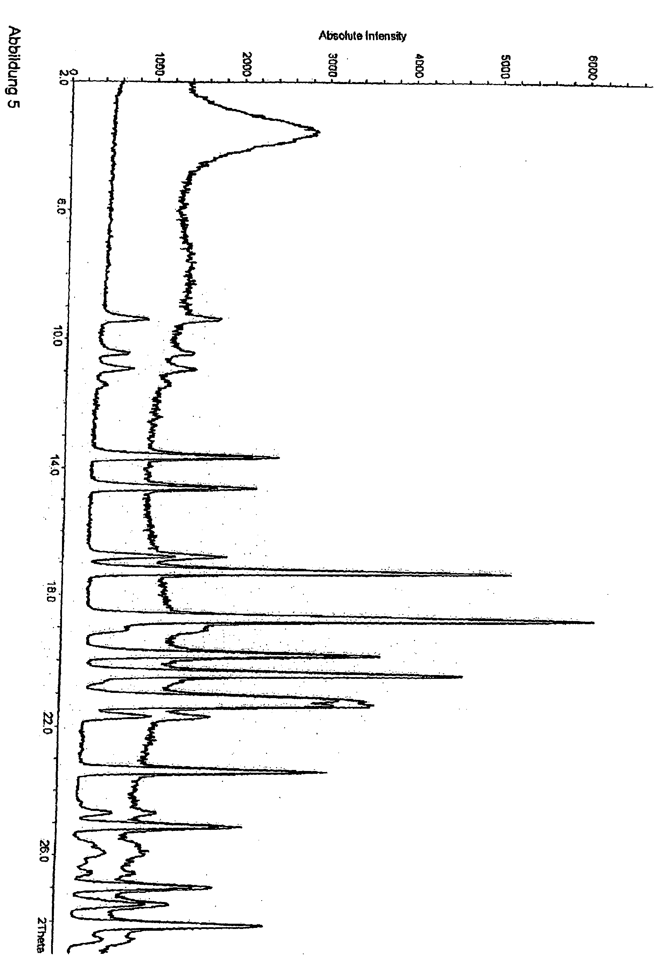

Wirkstoffgehalt des Absorbates mittels HPLC: 50,4% (theoretisch 50%)
Pulverröntgenbeugungsdiagramm: Abbildung 5
Verunreinigungsprofil (Summe aller Verunreinigungen, HPLC, in %):

| | **Ausgang** | **15 Tage (70°C / 75% relative Feuchtigkeit)** |
|---|---|---|
| **Probe (Adsorbat)** | **0,72** | **1,05** |
| **Vergleich (amorphes Atorvastatin-Calcium)** | **1,07** | **1,92** |

### Beispiel 6: Atorvastatin-Mikrokristalline Cellulose-Adsorbat

Zu einer Lösung von heterogenem Atorvastatin-Hemicalcium in Ethanol (0,15 g/ml) werden 0,30g/ml mikrokristalline Cellulose (Verhältnis Wirkstoff : Adsorbat 1:2) zugegeben und gleichmäßig suspendiert. Das Lösungsmittel wird nun unter permanenter Bewegung und Vakuum (Rotationsverdampfer oder Taumeltrockner) bei 25°C abgetrocknet. Die Mischung wird abschließend zur Entfernung von Restlösemittel kurzzeitig bei 35°C nachgetrocknet.

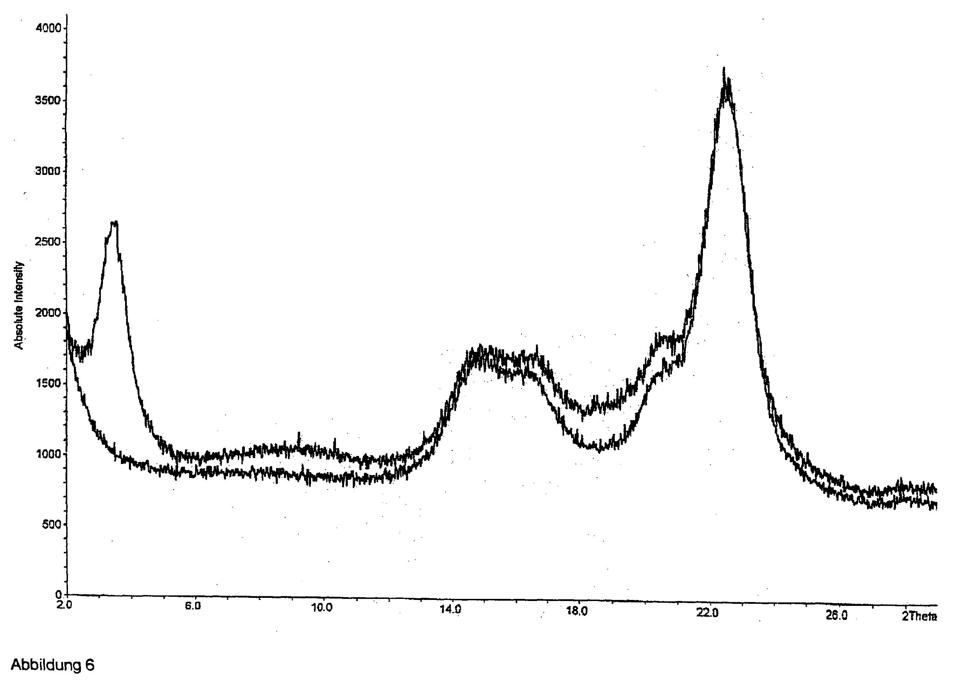

Wirkstoffgehalt des Absorbates mittels HPLC: 33,4% (theoretisch 33,3%) Pulverröntgenbeugungsdiagramm: Abbildung 6
Verunreinigungsprofil (Summe aller Verunreinigungen, HPLC, in %):

| | **Ausgang** | **15 Tage (70°C / 75% relative Feuchtigkeit)** |
|---|---|---|
| **Probe (Adsorbat)** | **0,91** | **1,09** |
| **Vergleich (amorphes Atorvastatin-Calcium)** | **1,07** | **1,92** |

## Patentansprüche

1. Pharmazeutische Formulierung in Form von den Wirkstoff schnell freisetzenden, durch Direktverpressung hergestellten Tabletten, **dadurch gekennzeichnet, dass** die Tabletten durch Direktverpressung von Atorvastatin-Adsorbaten herstellbar sind, wobei man von einer Lösung des Atorvastatins oder einem seiner Salze, Hydrate, Solvate, Ester, Lactone und Tautomere in wenigstens einem organischen Lösungsmittel mit einem Gesamtwassergehalt von nicht mehr als 10 Vol.-%, bevorzugt von nicht mehr als 5 Vol.-%, ausgeht, Adsorbermaterial, ausgewählt aus der Gruppe, bestehend aus Cellulosen, Cellulosederivaten, Polyolen, Zuckern, Zuckerderivaten, Maltodextrinen, Cyclodextrinen, Stärken, Polydextrosen oder Mischungen daraus, darin suspendiert und das Lösungsmittel durch Trocknen entfernt.

2. Pharmazeutische Formulierung gemäß Anspruch 1, **dadurch gekennzeichnet, dass** das Adsorbermaterial aus der Gruppe aus mikrokristalliner Cellulose, Lactose, Mannitol und Sorbit ausgewählt ist.

3. Pharmazeutische Formulierung gemäß Anspruch 1 oder 2, **dadurch gekennzeichnet, dass** die Atorvastatin-Adsorbate den Wirkstoff in fein verteilter, amorpher Form und gegebenenfalls als dessen Solvate enthalten.

4. Pharmazeutische Formulierung gemäß Anspruch 1 bis 3, **dadurch gekennzeichnet, dass** man ein Verhältnis von Wirkstoff zu Adsorbat im Bereich von 1 : 0,1 bis 10, insbesondere im Bereich von 1:1 bis 1:2 einstellt.

5. Pharmazeutische Formulierung gemäß einem oder mehreren der vorhergehenden Ansprüche 1 bis 4, **dadurch gekennzeichnet, dass** man als Lösungsmittel organische Lösungsmittel mit einem Gesamtwasseranteil von nicht mehr als 10-Vol.%, alleine oder in Mischungen einsetzt, wobei die organischen Lösungsmittel ausgewählt sind aus der Gruppe der niederen Alkanole mit 1 bis 4 Kohlenstoffatomen, der Gruppe der Ether und der Gruppe der aliphatischen Ketone und deren Gemischen.

6. Pharmazeutische Formulierung gemäß Anspruch 5, **dadurch gekennzeichnet, dass** man als Lösungsmittel Aceton, Ethanol, Methanol, Methylethylketon oder deren Gemische einsetzt.

7. Pharmazeutische Formulierung gemäß einem der Ansprüche 1 bis 6, **dadurch gekennzeichnet, dass** man eine Wirkstofflösung einsetzt, die im Rahmen der Atorvastatinsynthese anfällt und darin das Adsorbermaterial suspendiert.

8. Pharmazeutische Formulierung gemäß einem der Ansprüche 1 bis 7, **dadurch gekennzeichnet, dass** das Atorvastatin-Adsorbat den Wirkstoff in fein verteilter amorpher Form, bevorzugt als Alkalimetall- oder als Erdalkalimetallsalz sowie deren Hydrate und Solvate und besonders bevorzugt als Calciumsalz sowie dessen Hydrate enthält.

9. Pharmazeutische Formulierung gemäß einem der Ansprüche 1 bis 8, **dadurch gekennzeichnet, dass** sie weitere pharmazeutisch akzeptable Hilfsstoffe enthält.
